# EUROPEAN PATENT SPECIFICATION

(11) **EP 2 217 709 B1**
(45) Date of publication and mention of the grant of the patent: **05.11.2014**
(21) Application number: 08839475.4
(22) Date of filing: 15.10.2008
(51) Int. Cl.: C12N 15/85

(54) **GENETICALLY MODIFIED STEM CELLS AND METHODS FOR IDENTIFYING TISSUES DIFFERENTIATED THEREFROM**
GENTECHNISCH VERÄNDERTE STAMMZELLEN UND VERFAHREN ZUR IDENTIFIZIERUNG VON DARAUS DIFFERENZIERTEN GEWEBEN
CELLULES SOUCHES GÉNÉTIQUEMENT MODIFIÉES ET PROCÉDÉS D'IDENTIFICATION DE TISSUS DIFFÉRENCIÉS DE CES CELLULES SOUCHES

(30) Priority: 15.10.2007 HU 0700675
(43) Date of publication of application: 18.08.2010
(73) Proprietor: Orbán, Tamás, 1119 Budapest (HU); Izsvák, Zsuzsanna, 3200 Gyöngyös (HU); Német, Katalin, 1119 Budapest (HU); Apáti, Ágota, 1213 Budapest (HU); Sarkadi, Balázs, 1121 Budapest (HU)
(72) Inventor: Orbán, Tamás, 1119 Budapest (HU); Izsvák, Zsuzsanna, 3200 Gyöngyös (HU); Német, Katalin, 1119 Budapest (HU); Apáti, Ágota, 1213 Budapest (HU); Sarkadi, Balázs, 1121 Budapest (HU)
(74) Representative: Svingor, Adam
(86) International application number: PCT/IB2008/054238
(87) International publication number: WO 2009/050657

(56) References cited:
- WO-A-2006/017567
- XUE TIAN ET AL: "Functional integration of electrically active cardiac derivatives from genetically engineered human embryonic stem cells with quiescent recipient ventricular cardiomyocytes - Insights into the development of cell-based pacemakers" CIRCULATION, vol. 111, no. 1, 4 January 2005 (2005-01-04), pages 11-20, XP002525057 ISSN: 0009-7322
- MOORE J C ET AL: "Human embryonic stem cells: Genetic manipulation on the way to cardiac cell therapies" REPRODUCTIVE TOXICOLOGY, ELSEVIER SCIENCE, US, vol. 20, no. 3, 1 September 2005 (2005-09-01), pages 377-391, XP025333567 ISSN: 0890-6238 [retrieved on 2005-09-01]
- CARON L ET AL: "The Lac repressor provides a reversible gene expression system in undifferentiated and differentiated embryonic stem cell" CMLS CELLULAR AND MOLECULAR LIFE SCIENCES, BIRKHÄUSER-VERLAG, BA, vol. 62, no. 14, 1 July 2005 (2005-07-01), pages 1605-1612, XP019200786 ISSN: 1420-9071
- HONG SUNGHOI ET AL: "Functional analysis of various promoters in lentiviral vectors at different stages of in vitro differentiation of mouse embryonic stem cells." MOLECULAR THERAPY : THE JOURNAL OF THE AMERICAN SOCIETY OF GENE THERAPY SEP 2007, vol. 15, no. 9, September 2007 (2007-09), pages 1630-1639, XP002525058 ISSN: 1525-0016
- NIWA H ET AL: "EFFICIENT SELECTION FOR HIGH-EXPRESSION TRANSFECTANTS WITH A NOVEL EUKARYOTIC VECTOR" GENE, ELSEVIER, AMSTERDAM, NL, vol. 108, 1 January 1991 (1991-01-01), pages 193-200, XP000569330 ISSN: 0378-1119
- TAKEUCHI KOICHI ET AL: "Morphologic characterization of green fluorescent protein in embryonic, neonatal, and adult transgenic rats." ANATOMICAL RECORD, vol. 274A, no. 2, October 2003 (2003-10), pages 883-886, XP002525059 ISSN: 0003-276X
- ORBÁN TAMÁS I ET AL.: 'Applying a "double-feature" Promoter to Identify Cardiomyocytes Differentiated from Human Embryonic Stem Cells Following Transposon-Based Gene Delivery' STEM CELLS vol. 27, no. 5, 01 May 2009, pages 1077 - 1087, XP055037939 DOI: 10.1002/stem.45 ISSN: 1066-5099
- YOJI HAKAMATA ET AL: 'Green Fluorescent Protein-Transgenic Rat: A Tool for Organ Transplantation Research' BIOCHEMICAL AND BIOPHYSICAL RESEARCH COMMUNICATIONS vol. 286, no. 4, 01 August 2001, pages 779 - 785, XP055037970 DOI: 10.1006/bbrc.2001.5452 ISSN: 0006-291X

### SEQUENCE LISTING

<110> Tamás, ORBÁN
<120> Genetically modified stem cells and methods for identifying tissues differentiated therefrom
<130> 105010
<160> 1
<170> PatentIn version 3.5
<210> 1
   <211> 1132
   <212> DNA
   <213> Artificial Sequence
<220>
   <223> CAG fusion promoter
<400> 1
   ggccgctcta gcccctagtt attaatagta atcaattacg gggtcattag ttcatagccc      60
   atatatggag ttccgcgtta cataacttac ggtaaatggc ccgcctggct gaccgcccaa     120
   cgacccccgc ccattgacgt caataatgac gtatgttccc atagtaacgc caatagggac     180
   tttccattga cgtcaatggg tggagtattt acggtaaact gcccacttgg cagtacatca     240
   agtgtatcat atgccaagta cgccccctat tgacgtcaat gacggtaaat ggcccgcctg     300
   gcattatgcc cagtacatga ccttatggga ctttcctact tggcagtaca tctacgtatt     360
   agtcatcgct attaccatgg tcgaggtgag ccccacgttc tgcttcactc tccccatctc     420
   ccccccctcc ccacccccaa ttttgtattt atttattttt taattatttt gtgcagcgat     480
   gggggcgggg gggggggggg ggcgcgcgcc aggcggggcg gggcggggcg aggggcgggg     540
   cggggcgagg cggagaggtg cggcggcagc caatcagagc ggcgcgctcc gaaagtttcc     600
   ttttatggcg aggcggcggc ggcggcggcc ctataaaaag cgaagcgcgc ggcgggcggg     660
   agtcgctgcg ttgccttcgc cccgtgcccc gctccgcgcc gcctcgcgcc gcccgccccg     720
   gctctgactg accgcgttac tcccacaggt gagcgggcgg gacggccctt ctcctccggg     780
   ctgtaattag cgcttggttt aatgacggct tgtttctttt ctgtggctgc gtgaaagcct     840
   tgaggggctc cgggagggcc ccggcaggaa ggaaatgggc ggggagggcc ttcgtgcgtc     900
   gccgcgccgc cgtccccttc tccctctcca gcctcggggc tgtccgcggg gggacggctg     960
   ccttcggggg ggacggggca gggcggggtt cggcttctgg cgtgtgaccg gcggctctag    1020
   agcctctgct aaccatgttc atgccttctt ctttttccta cagctcctgg gcaacgtgct    1080
   ggttattgtg ctgtctcatc attttggcaa agaattgatt aattcgagcg aa            1132

## Description

### Field of the invention

This invention relates generally to genetically modified stem cells and to the selection of cells differentiated therefrom. Particularly, the herein disclosed invention relates to stem cells or cells differentiated therefrom containing a copy of a stably inheritable expression construct that is suitable for the expression of transgenes in stem cells, wherein said construct comprises at least a a CAG promoter wherein the nucleotide sequence of said CAG promoter is the sequence given in SEQ ID NO:1, said promoter being operable both in stem cells and in differentiated tissues, wherein said promoter is constitutive, while the expression level thereof being subject to a tissue or cell type specific regulation in cardiomyocytes, and under the control of said promoter, a transgene, wherein said transgene is expressed in the stem cell. Furthermore methods are disclosed to produce such stem cells, as well as specific uses of said stem cells in assay methods and in human therapy and in veterinary practice.

### Background of the invention

The application of stem cells provides new hopes in the clinical treatment of a number of diseases and, at the same time, they are excellent models for tissue development and physiological cell differentiation. The so called "regenerative medicine" makes use of cells that can grow and differentiate to replace a damaged tissue. Also, efficient and stable gene delivery into stem cells should form the basis of successful gene therapy applications. Human embryonic stem (HuES) cells represent a great potential for gene therapy purposes. Nevertheless, one of the major difficulties of HuES-based applications is to achieve efficient, stable gene delivery and to conduct a directed tissue differentiation and separation. This would undoubtedly provide useful tools in medical research and practice, providing large quantities of reproducible model systems for various medically relevant diseases.

Current methods for directed tissue differentiation often apply endogenous morphogenetic proteins or invasive chemicals on HuES cells to obtain the tissue(s) of interest (Lev, S et al., Ann N Y Acad Sci 2005, 1047:50; Karner, E et al., Stem Cells Dev 2007, 16:39; Chen, K et al., J Cell Biochem 2008, 104:119). However, the use of such artificial cocktail of drugs is far from natural differentiation and, among other problems, could probably induce undesired gene expression profiles. In addition, it is difficult to prove that tissues derived from such invasive attempts reliably represent naturally occurring cell types (Tomescot, A et al., Stem Cells 2007, 25:2200).

Currently, the most widely applied methods for gene delivery into various stem/progenitor cells are based on the use of viral vector constructs. By now, there are numerous efficient retrovirus- lentivirus- or other virus-based methods which allow stable genomic incorporation of the foreign DNAs with high gene product expression levels (see e.g. WO 06126972). However, virus-based gene therapy technologies also have serious drawbacks, including safety concerns of virus production, and the preferential insertion of virus into active genes, which might cause uncontrolled proliferation of the gene-modified stem cells (Schroder, AR et al., Cell 2002, 110:521; VandenDriessche, T et al., Curr Gene Ther 2003, 3:501).

A common way of overcoming the above problems is to use a combination of tissue-specific promoters and certain drug resistance genes as selection markers. This method can provide the solution for obtaining the desired cell types since it is based on "spontaneous" differentiation of HuES cells (at least without the induction of invasive chemicals, mimicking more natural conditions, see Passier, R et al., Stem Cells 2005, 23:772) and selecting for the differentiated cell types in a latter phase, eg. selecting by certain antibiotics (Kolossov, E et al., J Exp Med 2006, 203:2315; Huber, I et al., FASEB J 2007, 21:2551). In principle, this could result in the enrichment of desired tissues in large quantities and most likely providing less interference with the gene expression profiles of the tissues of interest. Although better than using artificial chemicals, it requires viral or non-viral gene delivery into HuES cells which itself can be technically challenging, and antibiotic selection could also induce a certain way of differentiation, again asking for the need to prove that the obtained tissues represent naturally occurring cell types (Duan, Y et al., Stem Cells 2007, 25:3058).

The usage of tissue specific promoters generates another technical problem. When using a promoter with high tissue specificity (which is certainly required by this application), the transgene expression will not be detectable in undifferentiated HuES cells (Gallo, P et al., Gene Ther 2008, 15:161). Especially for non-viral applications, where the efficiency of delivery is generally quite low, this represents a serious disadvantage. To avoid this, one can use another constitutive promoter to drive the expression of an additional selection marker gene to first select for transgene delivery and expression, then after spontaneous differentiation, for the forming of specific tissues. Apart from being cumbersome and time-consuming, this also creates another technically challenging point since it either means the co-delivery of different transgenes (eg. co-transfection with two plasmids) or a delivery of a larger genetic cargo, often resulting in lower delivery efficiency.

Caron L. et al. (Cell. Mol. Life. Sci. 2005, 62 1605) established embryonic stem cell lines stably expressing reporter genes under inducible control and found a five- to tenfold IPTG induction of transgene expression. They used a CAG promoter comprising a chicken beta-actin promoter and a CMV enhancer which was modified by inserting a LacI-binding site so as to allow IPTG control of the promoter. The regulatable expression was maintained throughout the differentiation process of the ES cells. While they could control regulation it did not depend on differentiation status or tissue type.

The solution for the above stated problems could be a promoter that is operable in stem cells and at the same time has tissue specific regulation in differentiated cells. Such a promoter would have many advantages, by using such a promoter there would be no need for using viruses and the selection of differentiated cells could be done with the same construct. An ideal solution would be a constitutive promoter that is operable both in stem cells and in differentiated tissues, the expression level thereof being subject to a tissue or cell type specific regulation in differentiated cells. Such a promoter would enable the expression of an introduced transgene that could be expressed in the stem cell or the cell differentiated therefrom.

Therefore the object of the present invention was to develop an application of a non-viral methodology for efficient and stable gene delivery into stem cells, preferably into HuES cells. In the detailed description below, we shall demonstrate several examples concerning stem cells, the cells differentiated from said stem cells and the methods according to the present invention, etc. However it is obvious for a person skilled in the art that herein only certain specific embodiments of the present invention are described to assist the person skilled in the pertinent art. Clearly, we have no intention to limit the scope of the present invention with the described examples, they are only assisting in the use of the present invention.

The term "double-feature" constitutive promoter within the context of this invention refers to a promoter that enables constitutive expression in stem cells, but at the same time drives expression differently in different types of differentiated cells or tissues.

### Summary of the invention

The technical solution according to the present invention is based on the surprising and unexpected finding, that certain promoters, like the CAG promoter the sequence given of which is given in SEQ ID NO:1 is capable of driving a constitutive expression in stem cells while at the same time the expression driven by said promoter is tissue or cell type specific in differentiated cells, specifically in cardiomyocytes, therefore the said promoter is called a double-feature constitutive promoter. Particularly the expression of the CAG promoter was found to be extremely strong in cardiomyocytes. This unexpected behavior in itself enables the selection of differentiated cardiomyocytes.

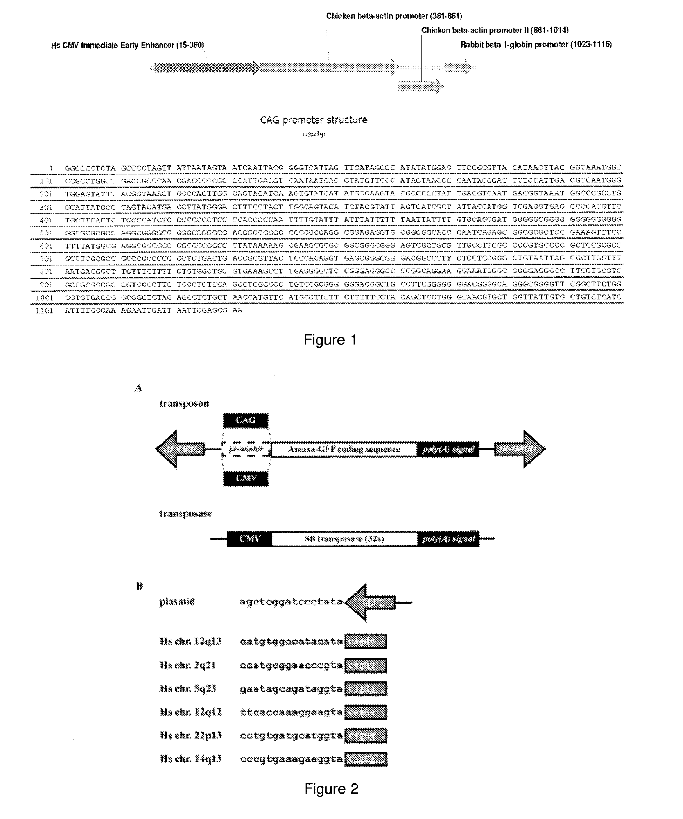

The term "CAG promoter" refers herein to a promoter that is a fusion promoter and generally contains the Cytomegalovirus (CMV) Immediate Early Enhacer, two parts of the chicken beta-actin promoter and part of the rabbit beta1-globin promoter (see fig. 1). More specifically, in the 1132 bp long promoter constructs, nucleotide region 15-380 corresponds to the CMV Immediate Early Enhancer, nucleotide regions 381-861 and 861-1014 correspond to the two parts of the chicken beta-actin promoter sequences, and nucleotide region 1023-1126 represents the rabbit beta1-globin promoter sequence. Other short sequences on the construct represent linker sequences (Niwa, H et al., Gene 1991, 108:193). According to the present invention the CAG promoter's sequence is the sequence given in SEQ ID NO:1.

### Detailed description of the invention

The present invention relates to animal or human stem cells or cells differentiated therefrom containing a copy of a stably inheritable expression construct that is suitable for the expression of trans genes in stem cells, said construct comprising:
- at least a a CAG promoter wherein the nucleotide sequence of said CAG promoter is the sequence given in SEQ ID NO:1, said promoter being operable both in stem cells and in differentiated tissues, wherein said promoter is constitutive, while the expression level thereof being subject to a tissue or cell type specific regulation in cardiomyocytes, and
- under the control of said promoter, a transgene, wherein said transgene is expressed in the stem cell.

According to a preferred embodiment, the above expression construct further comprises viral transduction or transposon derived sequences.

According to a still further preferred embodiment of the invention the above expression construct further comprises a pair of sequences allowing site-specific recombination upstream of the promoter and downstream of said transgene and, preferably, the stem cell comprises a further construct enabling the expression of
- a site specific recombinase and
- a further transgene controlled by a promoter flanked by a pair of sequences allowing site-specific recombination.

Preferably said pair of sequences allowing site-specific recombination are the Lox sites of the Cre recombinase or the FRT sites of the FLP recombinase, and the site-specific recombinase is the Cre recombinase or the FLP recombinase, respectively.

According to a preferred embodiment of the present invention said transgene is a reporter gene and a reporter protein encoded by said reporter gene is expressed in said stem cell, wherein preferably said reporter protein gives rise to a detectable signal the intensity of which correlates with the activity of said promoter or said reporter protein gives specific resistance to a chemical agent which resistance correlates with the activity of said promoter. A person skilled in the art would recognize that the specific function of said transgene is not to be construed as limiting to the present invention as long as it can be expressed in stem cells. Therefore said transgene can be a construct for siRNA expression and a connected reporter gene, and the siRNA and the reporter protein encoded by said reporter gene are expressed in said stem cell or in its differentiated derivative, wherein preferably said reporter protein gives a detectable signal the intensity of which correlates with the activity of said promoter. However, preferably, said reporter protein is a fluorescent protein selected from a group comprising Green Fluorescent Protein (GFP), Red Fluorescent Protein (RFP), Yellow Fluorescent Protein (YFP), Cyan Fluorescent Protein (CFP), DS-redMST Fluorescent Protein; Luciferase; β-galactosidase; or a protein providing resistance to chemical selecting agents, advantageously to puromycin, neomycin or other cytotoxic agent.

The stem cell of the invention is an animal or a human stem cell. Furthermore said stem cell can be animal or human embryonic stem cell. Preferably the stem cell of the invention is a human embryonic stem cell (HUES).

The invention further concerns a cell differentiated from the stem cell of the invention, wherein said stem cell comprises a stably inherited expression construct that is suitable for expression of a transgene in stem cells, said construct comprising at least the above defined constitutive promoter which is operable in stem cells, wherein the expression level of said promoter is subject to a tissue or cell type specific regulation, and, optionally, a transgene under the control of said promoter.

The invention also relates to a method for producing a stem cell or a differentiated stem cell according to the invention, comprising the following steps:
i) providing a construct that comprises
   - at least a CAG promoter wherein the nucleotide sequence of said CAG promoter is the sequence given in SEQ ID NO:1, said promoter being operable both in stem cells and in differentiated tissues, wherein said promoter is constitutive, while having an expression level being subject to a tissue or cell type specific regulation in cardiomyocytes, and, optionally,
   - under the control of said promoter, a transgene, wherein said transgene is expressed in said stem cell
ii) introducing the construct as defined in step i) into a stem cell and, optionally,
iii) differentiating said stem cell.

According to a preferred embodiment said introduction of said construct in step ii) is done by viral transduction or by using a transposon - transposase system. The used transposon - transposase system could be the well known *Sleeping Beauty* or the newly discovered *Frog Prince* transposon - transposase system (for the description of the latter see e.g. EP1507865). The above-mentioned expression construct may further comprise a pair of sites allowing site-specific recombination upstream of said promoter and downstream of said transgene and, preferably, said stem cell comprises a further vector comprising
i) a pair of sites allowing site-specific recombination and
ii) a further transgene under the control of a promoter, flanked by sites allowing site-specific recombination. The further transgene can be any gene that is operable in stem cells or in cells differentiated therefrom, while it is an object of the invention to provide a stem cell into which any transgenes can be easily incorporated.

In a still further preferred embodiment of the invention said expression construct further comprises a pair of sites allowing site-specific recombination upstream of said promoter and downstream of said transgene and, preferably, said stem cell comprises a further construct comprising
i) a pair of sites allowing site-specific recombination and
ii) a construct allowing siRNA expression and connected reporter gene expression flanked by sites allowing site-specific recombination.

Said transgene or further transgene can be a reporter gene and a reporter protein encoded by said reporter gene is expressed in said stem cell or differentiated cell, wherein preferably said reporter protein gives a detectable signal the intensity of which correlates with the activity of the promoter. Preferably said reporter protein is a fluorescent protein selected from a group comprising Green Fluorescent Protein (GFP), Red Fluorescent Protein (RFP), Yellow Fluorescent Protein (YFP), Cyan Fluorescent Protein (CFP), DS-redMST Fluorescent Protein; Luciferase; β-galactosidase; or a protein providing resistance to chemical selecting agents, advantageously to puromycin or neomycin.

The invention further concerns the use of stem cells according to the invention for testing the effect of a test compound on stem cell differentiation.

A method of identifying or profiling a modulator of cell differentiation is also disclosed, said method comprising:
i) contacting a test sample comprising stem cells according to the invention with a test compound, including drugs, hormones, artificial or natural compounds, and
ii) allowing said stem cells to differentiate;
iii) determining the effect of said test compound on the amount of said reporter gene product or activity in the differentiated cells as compared to a control sample.

There is also disclosed a method for monitoring stem cell differentiation comprising:
i) providing a stem cell of the present invention,
ii) monitoring the expression level of said reporter gene or the amount or activity of said reporter gene product, and
iii) drawing conclusions regarding the direction of the differentiation of said stem cell based on the expression level of said reporter gene in any of the cells differentiated from said stem cell.

Said monitoring can be done by measuring the intensity of the signal emitted by the product of said reporter gene.

The invention further concerns a reagent kit comprising:
- a stem cell of the present invention, and
- one or more test reagents to implement any of the above said methods.

The above method is especially suited to test drugs affecting cardiomyocyte differentiation, as any effect that influences this pathway can be monitored and quantified by exploiting the unique feature of the CAG promoter. Also, the effect of drugs on any other promoter during the above mentioned differentiation pathway may also be examined since cardiomyocytes or their progenitor cells can be studied selectively. In addition, the CAG promoter driven gene expression-based enrichment provides opportunities for high-throughput pharmacological testing of differentiated cardiomyocytes.

The stem cell or a cell differentiated therefrom according to the invention or a stem cell produced according to a method of the invention can be used in human or veterinary therapy. The stem cell or a cell differentiated therefrom has a great advantage compared to other stem cells, namely it can be selected without disturbing said stem cell or a cell differentiated therefrom with compounds (e.g. antibiotics or other selecting agents) normally used for the selection of cells. A further advantage in this respect is that the stem cells or the cells differentiated therefrom can be produced without using viruses. This is advantageous since the use of viruses bears an inherent health hazard. The CAG promoter's surprising double-feature behavior, namely it is constitutive in stem cells, but at the same time its expression is extremely strong in cardiomyocytes is a very unique property. Other constitutive promoters (e.g. EF1α, PGK and CMV) were also tested in this respect but these promoters did not show the above double-feature property. Furthermore this behavior is independent of the transgene sequence, as amaxaGFP and EGFP gave the same results as the canonical GFP. Besides, this behavior is independent of the gene delivery method as transposon-based and lentiviral-based methods gave the same results. The exact integration site of the promoter also does not affect this surprising behavior. Moreover this behavior is independent of the transgene copy number.

### Brief description of the drawings

Fig. 1 shows the general structure of the CAG promoter and its nucleotide sequence.

On Fig. 2 transposons and integration sites are depicted. (A) Structure of the used transposons and SB transposase expressing plasmids. The transposon constructs contained either the CAG or the CMV promoters whereas the transposase was only expressed transiently by the CMV promoter. (B) Examples of transposon integration sites in HUES cells determined by splinkerette or inverse PCR; Hs chr.= Homo sapiens chromosome.

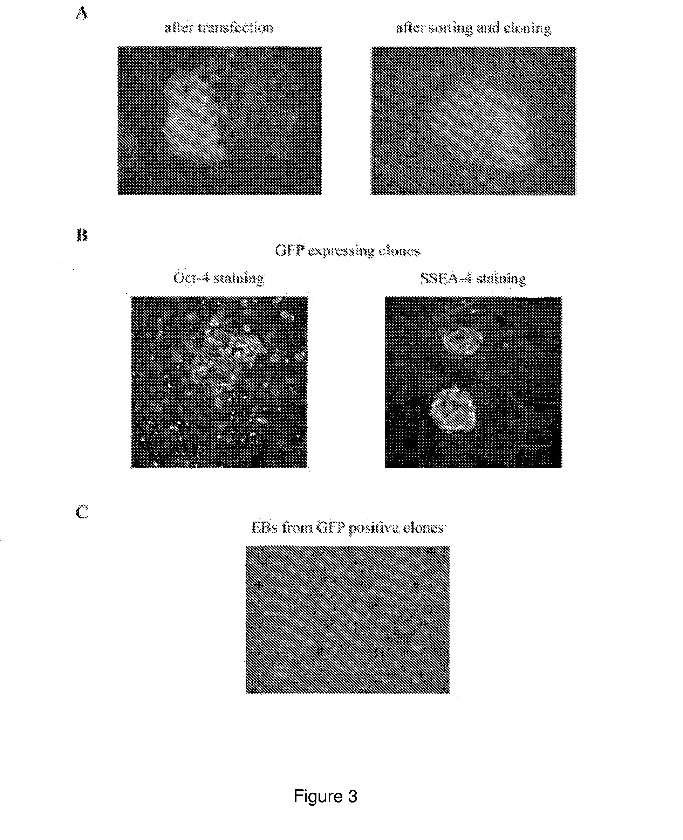

Fig. 3 shows human embryonic stem cells stably expressing the GFP transgene. (A) Morphology and GFP expression of human embryonic stem cell (HUES9) clumps. GFP expression detected by a fluorescent light microscope following transfection (left panel), or after subsequent sorting and cloning of the transgene expressing cells (right panel - magnification: 200x). (B) GFP expressing HUES9 clones on mouse embryonic fibroblasts stained for embryonic stem cell markers. The Oct-4 transcription factor is localized in the nucleus, whereas the SSEA-4 protein is localized in the plasma membrane. Merged confocal images, sizes indicated by the scale bars. Blue: Oct-4 (left panel) or SSEA-4 protein (right panel); green: GFP; red: Tric-phalloidin, representing actin filaments; white: DAPI staining for the nucleus in the case of Oct-4 detection. (C) Six days old EBs formed from GFP expressing HUES9 clones in fluorescence microscopy (magnification: 40x).

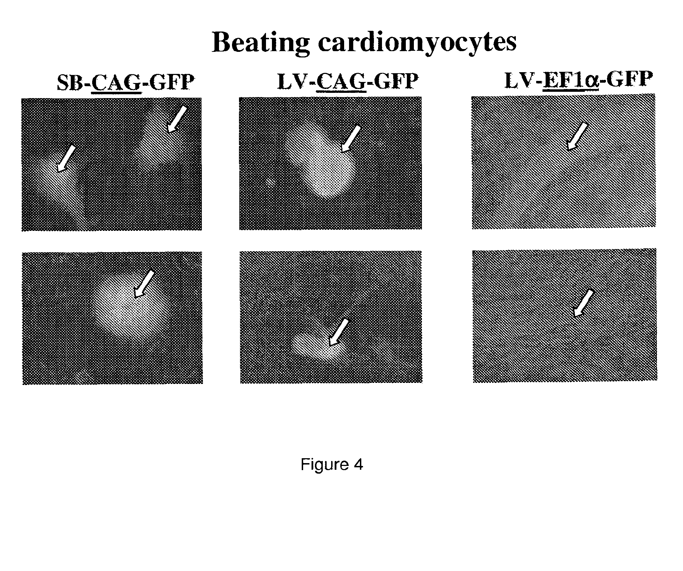

Fig. 4 shows a typical area of differentiated HUES9 cells with beating cardiomyocytes (white arrows). The pictures represent the prominent difference of GFP expression between the cardiomyocytes and surrounding other tissues (40x magnification). For the CAG promoter, the epxression intensity of GFP is always several magnitudes higher in beating cardiomyocytes than in the surrounding other tissue types. This phenomenon is independent of the gene delivery method as transposon-based transgenes (SB-Sleeping Beauty transposon) or lentiviral(LV)-derived transgenes behave similarly. However, this phenomenon cannot be seen when using other constitutive promoters (such as the EF1α promoter). White arrows point to beating cardiomyocytes differentiated from clones of transgenic human embryonic stem cells. The simplified structure of the transgene cassette is indicated on the top of the pictures.

### EXAMPLES

The following examples further illustrate the present invention but should not be construed as in any way limiting its scope.

### Reference Example 1

### HUES9 cell culturing and differentiation

The human embryonic stem cell line HUES9 was maintained according to the widely accepted culture protocol described in Cowan, CA et al., N Engl J Med 2004, 350:1353. Briefly, cells were cultured on mitomycin-C treated mouse embryonic fibroblast feeder cells in complete HUES medium consisting of 15% knockout serum replacement (Gibco, Grand Island, NY), 80% knockout Dulbecco modified Eagle medium (KoDMEM) medium (Invitrogen, Carlsbad, CA), 1 mM L-glutamine, 0.1 mM beta-mercaptoethanol, 1% nonessential amino acids, and 4 ng/mL human fibroblast growth factor. HUES cells from passage no. 35 were used for these analyses.

The differentiation of the HUES cells were initiated spontaneously. On the day of passage, undifferentiated cells at confluence in 6-well plates were treated with collagenase IV and were transferred to Poly(2 Hydroxyethyl-methacrylate) (Sigma-Aldrich, St. Louis, MO) treated Petri dishes to allow EB formation. Cells were kept in differentiation medium consisting of KoDMEM supplemented with 20% non heat-inactivated fetal bovine serum (Invitrogen, Carlsbad, CA), 1% nonessential amino acids, 1 mM L-glutamine, and 0.1 mM beta-mercaptoethanol for six days, the medium being changed daily. Under these conditions, the HUES cells generate EBs which are complex, teratoma-like tissue structures with highly variable forms and tissue elements (Lev, S et al., Ann N Y Acad Sci 2005, 1047:50; Thomson, JA et al., Science 1998, 282:1145). These EBs were placed onto gelatin-coated plates, where they attach to the surface and continue a further spontaneous differentiation process by forming recognizable tissue-types. Under these conditions, formation of endothelial, epithelial, and neuronal cells, as well as of fibroblasts and cardiomyocytes could be observed. In this study we applied conditions to provide an enrichment of cardiomyocytes and/or neural precursors (so called rosettes) and differentiated neurons, in addition to the generally appearing fibroblasts in the culture plates (Schulz, TC et al., BMC Neurosci 2003, 4:27). Cell types were identified by morphological signs under phase contrast light microscopy, as well as by immunostaining for various protein markers (see below, Example 5). For puromycin selection (see also Example 2), 0.8 µg/ml puromycin was used for undifferentiated HuES cells.

### Example 2

### Transposon constructs and transfection procedure

SB transposon plasmids used in this project contained the cDNA of the highly fluorescent marker Amaxa-GFP (www.amaxa.com) (Fig. 2A) or the canonical EGFP between the transposon inverted repeat sequences. Promoter sequences can be obtained from a tissue-specific promoter database, e.g. the TiProD: Tissue-Specific Promoter Database, (http://tiprod.cbi.pku.edu.cn:8080/index.html), including cardiac actin or human albumin promters. Other chemically inducible promoters, e.g. a metallothionein promoter, or artificial fusion promoters, eg. the CAG promoter can also be utilized. The CAG is a composite promoter that combines the human cytomegalovirus immediate-early enhancer, two parts of the chicken beta-actin promoter, and one part of the rabbit beta1-globin promoter. The CAG promoter is a very strong and ubiquitous promoter. It produces high levels of expression both in vitro and in vivo. The CAG promoter (SEQ ID NO:1) was successfully used to express the enhanced GFP in all tissues of transgenic mice with the exception of erythrocytes and hair. Comparison analyses have shown that the CAG promoter is more efficient than the CMV promoter/enhancer.

By a HindIII and NheI restriction digestion followed by ligation, we cloned the CMV viral promoter, the CAG promoter (Fig. 2A), the human phosphoglycerate kinase (PGK) promoter or the short version of the human elongation factor 1α (EF1α) promoter upstream of the transgene. For the antibiotic selection experiments, the puromycin resistance gene was cloned between the transposon inverted repeat sequences driven by the CAG promoter. For the SB transposase, we used an enhanced version of the protein having 32 times higher transposase activity than the originally reconstructed species [unpublished results]; for transposition control, we applied a DDE mutant form of the protein (Ivics, Z et al., Cell 1997, 91:501). For transfection, the FuGENE^{®} 6 (Roche Applied Science, Switzerland) reagent was used according to the manufacturer's instruction. Before transfection, HUES cells were separated from the mouse feeder cells and placed on gelatin-coated plates. The subsequent day, the cells were co-transfected with transposon and transposase plasmids in a 10:1 ratio to avoid the overproduction inhibition of the transposase (Izsvak, Z et al., Mol Ther 2004, 9:147; Izsvak, Z et al., J Mol Biol 2000, 302:93). Next day, the transfected HUES cells were placed back onto the mouse feeder layer to keep them in an undifferentiated state.

### Example 3

### Detecting transposon activity and determining transposon integration sites

To provide evidence that the transposon construct is capable of transposition, the "excision PCR", a nested PCR method was applied, as described previously. This method amplifies the "footprint" sequence left on the plasmid after transposition, whereas no PCR product is obtained if no transposition occurs (Ivics, Z et al., Mol Ther 2007, 15:1137). To determine the integration sites of the transgenes in human genomic DNA, splinkerette PCR and inverse PCR methods were applied, essentially as described earlier (Ivics, Z et al., Cell 1997, 91:501; Vigdal, TJ et al., J Mol Biol 2002, 323:441). Briefly, for the splinkerette PCR, either a Sau3AI restriction enzyme, or the BfaI/NdeI/AseI enzyme cocktail was used to digest the genomic DNA, followed by a nested PCR using primers described earlier (Ivics, Z et al., Cell 1997, 91:501; Vigdal, TJ et al., J Mol Biol 2002, 323:441). For the inverse PCR, the BamHI/BclI enzyme cocktail was applied for the digestion of the genomic DNA, followed by a nested PCR using previously described primer pairs (Ivics, Z et al., Cell 1997, 91:501; Vigdal, TJ et al., J Mol Biol 2002, 323:441).

### Example 4

### Lentiviral constructs and transduction procedures

For the viral-based gene delivery, the new generation SEW lentiviral vectors were used (Schambach, A et al., Mol Ther 2007, 15:1167). The transgenes used for the experiments were exactly the same as used for the transposon constructs (see Example 2 above), each case removing the transcription unit from the transposon vector by restriction digestion and inserting it into the viral vector by blunt end ligation. Virus titers and transduction procedures were made and performed essentially as described previously (Ujhelly, O et al., Hum Gene Ther 2003, 14:403).

### Example 5

### Flow cytometry

Prior to cell sorting, GFP-expressing undifferentiated HUES cell colonies were manually selected by using a phase contrast light microscope under sterile conditions, to enrich the marker gene expressing population. Such heterogeneous colonies (see Fig. 3A) were harvested from the mouse feeder cells, washed with 1xPBS and sorted based on GFP fluorescence using the FACS Aria High Speed Cell Sorter (Beckton-Dickinson, San Jose, CA). Mock-transfected HUES cells were measured to set the level for GFP-positivity with the FACS Diva analysis software; propidium iodide staining was used to gate for the non-viable cells during the sorting procedure. Sorted GFP positive single cells were placed onto the mouse feeder cells and then monitored until the formation of surviving clones. For further gene expression analysis, differentiated cells from GFP-expressing HuES clones were sorted into 4 different artificial fractions based on decreasing GFP fluorescent signal intensity. Cells obtained from different fractions were washed with 1xPBS and immediately resuspended in Trizol^{®} (Invitrogen) for further RNA profile analysis (see Example 7 below).

### Example 6

### Immunohistochemical assays

For immunostaining, cells were seeded onto eight-well Nunc Lab-Tek II Chambered Coverglass (Nalge Nunc International, Rochester, NY). Cells were fixed with 4% paraformaldehyde in Dulbecco's modified PBS (DPBS) for 15 min at room temperature. Following further washing steps with DPBS, the cells were blocked for 1 hr at room temperature in DPBS containing 2 mg/ml bovine serum albumin, 1% fish gelatin, 0.1% Triton-X 100, and 5% goat serum. The samples were then incubated for 1 hr at room temperature with monoclonal antibodies for stem cell markers Oct-4 (1:50 dilution, Santa Cruz Biotechnology, Santa Cruz, CA), SSEA-4 (1:10 dilution, R&D Systems, Minneapolis, MN) or podocalyxin (1:10, R&D Systems, Minneapolis, MN). Incubations were also carried out using antibodies against the neuron-specific beta III tubulin (1:200 dilution, R&D Systems, Minneapolis, MN). After washing with DPBS, the cells were incubated for 1 hr at room temperature with Alexa Fluor 647-conjugated goat anti-mouse IgG antibody. Tric-phalloidin (Sigma-Aldrich, St. Louis, MO) staining was used for the detection of actin filaments, in the final concentration of 0.1 µg/ml. DAPI (Invitrogen, Madison, WI) was used for nuclear staining, GFP was evaluated by direct fluorescence. The stained samples were studied by an Olympus FV500-IX confocal laser scanning. The blue, green and deep red fluorescence were acquired between 430-460 nm, 505-525 nm, and above 660 nm; using 405 nm, 488 nm, and 633 nm excitations, respectively. All documented measurements were carried out on cells from four independent GFP-expressing clones, at least in triplicate stainings.

### Example 7

### RNA analysis

RNA isolation was carried out from cells collected in Trizol^{®} reagent (Invitrogen) according to the manufacturer's instruction. cDNA samples were prepared from 0.1 µg total RNA using the Promega Reverse Transcription System Kit as specified by the manufacturer. Tissue specific genes were selected using the TiProD tissue-specific promoter database (see Example 2 above). The following markers were selected: OCT4 and NANOG transcripts as undifferentiated stem cell markers; ACTC, NPPA and PLN genes as cardiac specific markers; PAX6 gene as an early marker for neuronal differentiation; CAPG gene as a skin differentiation marker; P0 ribosomal protein and Pol IIA genes as endogenous controls. Pre-developed real-time TaqMan^{®} assays for the listed genes were purchased from Applied Biosystems. For quantifying EFGP mRNA, specific TaqMan^{®} assay were designed for the cDNA sequence. Real-time PCR analyses were carried out using the StepOne™ Real-Time PCR System (Applied Biosystems), according to the manufacturer's instructions.

## Claims

1. An animal or human stem cell containing a copy of a stably inheritable expression construct that is suitable for the expression of transgenes in stem cells, said construct comprising:
- at least a CAG promoter wherein the nucleotide sequence of said CAG promoter is the sequence given in SEQ ID NO:1, said promoter being operable both in stem cells and in differentiated tissues, wherein said promoter is constitutive while
the expression level thereof being subject to a tissue or cell type specific regulation in cardiomyocytes, and,
- under the control of said promoter, a transgene, wherein said transgene is expressed in said stem cell.

2. The stem cell according to claim 1, which is a human embryonic stem cell (HUES).

3. The stem cell according to claim 1, which is an animal embryonic stem cell.

4. The stem cell according to any preceding claim, wherein said expression construct further comprises sequences selected from the following group:
- viral transduction sequences,
- transposon derived sequences,
- a pair of sequences allowing site-specific recombination upstream of the promoter and downstream of said transgene.

5. The stem cell according to any preceding claim, wherein said transgene is selected from
- a reporter gene and a reporter protein encoded by said reporter gene is expressed in said stem cell,
- a construct for the expression of a siRNA and a connected reporter gene, and said siRNA and the reporter protein encoded by said reporter gene are expressed in said stem cell or in its differentiated derivative,
- a gene encoding for a fluorescent protein selected from a group comprising Green Fluorescent Protein (GFP), Red Fluorescent Protein (RFP), Yellow Fluorescent Protein (YFP), Cyan Fluorescent Protein (CFP), DS-redMST Fluorescent Protein; Luciferase; β-galactosidase; and
a gene encoding for a protein providing resistance to chemical selecting agents, preferably to puromycin, neomycin or other cytotoxic agents.

6. A cell differentiated from the stem cell according to any preceding claim, wherein said cell comprises a stably inherited expression construct that is suitable for expression of a transgene in stem cells, said construct comprising
- at least a CAG promoter wherein the nucleotide sequence of said CAG promoter is the sequence given in SEQ ID NO:1, said promoter being operable both in stem cells and in differentiated tissues, wherein said promoter is constitutive while
the expression level thereof being subject to a tissue or cell type specific regulation in cardiomyocytes, and,
a transgene under the control of said promoter, wherein said transgene is expressed in said stem cell.

7. The differentiated cell according to claim 6, wherein said cell is a cardiomyocyte.

8. Method for producing a differentiated stem cell *in vitro,*
said differentiated stem cell comprising
a stably inheritable expression construct that is suitable for expression of a transgene in stem cells, said construct comprising
- at least the double-feature CAG promoter as defined in claim 1 and,
- a transgene under the control of said promoter;
said method comprising
i) providing a construct that comprises
- at least the CAG promoter as defined in claim 1, and,
- under the control of said promoter, a transgene, wherein said transgene is expressed in said stem cell,
ii) introducing the construct defined in step i) into an animal or human stem cell and,
iii) differentiating said stem cell.

9. The method of claim 8, wherein
- said introduction of said construct in step ii) is done by viral transduction or by using a transposon - transposase system.

10. Use of stem cells according to any one of claims 1 to 5 for testing the effect of a test compound on stem cell differentiation.

11. A reagent kit comprising:
- a stem cell defined in any of claims 1 to 5, and
- one or more test reagents enabling the implementation of the use according to claim 10.

## Patentansprüche

1. Tierische oder menschliche Stammzelle enthaltend eine Kopie eines dauerhaft vererbbaren Expressionskonstrukts, das für die Expression von Transgenen in Stammzellen geeignet ist, wobei das Konstrukt umfasst:
- mindestens einen CAG-Promotor, wobei die Nukleotid-Sequenz des CAG-Promotors der in der SEQ ID Nr. 1 gegebenen Sequenz entspricht, und der Promotor sowohl in Stammzellen als auch in differenzierten Geweben funktionsfähig ist, wobei der Promotor konstitutiv ist während die Expressions-Ebene hiervon einer Geweberegulation oder zelltypspezifischen Regulation in Kardiomyozyten unterliegt, und
- ein Transgen, das unter der Kontrolle des Promotors ist, wobei das Transgen in der Stammzelle exprimiert wird.

2. Stammzelle nach Anspruch 1, die eine menschliche embryonale Stammzelle (HUES) ist.

3. Stammzelle nach Anspruch 1, die eine tierische embryonale Stammzelle ist.

4. Stammzelle nach einem der vorhergehenden Ansprüche, wobei das Expressionskonstrukt des Weiteren Sequenzen umfasst, die aus der folgenden Gruppe ausgewählt sind:
- Virale Transduktionssequenzen,
- von Transposonen abgeleitete Sequenzen,
- ein Paar von Sequenzen, das eine seitenspezifische Rekombination strangaufwärts des Promotors und strangabwärts des Transgens erlaubt.

5. Stammzelle nach einem der vorhergehenden Ansprüche, wobei das Transgen ausgewählt ist aus:
- Einem Reportergen, und ein Reporterprotein, das durch das Reportergen kodiert ist, wird in der Stammzelle exprimiert,
- einem Konstrukt für die Expression einer siRNA und einem angekoppelten Reportergen, und die siRNA und das Reporterprotein, das durch das Reportergen kodiert wird, werden in der Stammzelle oder in deren differenziertem Derivat exprimiert,
- einem Gen, das für ein fluoreszierendes Protein kodiert, das ausgewählt ist aus einer Gruppe umfassend grün fluoreszierendes Protein (GFP), rot fluoreszierendes Protein (RFP), gelb fluoreszierendes Protein (YFP), cyan fluoreszierendes Protein (CFP), DS-redMST fluoreszierendes Protein; Luziferase; β-Galactosidase; und
- einem Gen, das für ein Protein kodiert, das Widerstandsfähigkeit gegen chemische Selektionsmittel, vorzugsweise gegen Puromycin, Neomycin und andere zytotoxische Mittel, verleiht.

6. Zelle, die von der Stammzelle gemäß einem der vorhergehenden Ansprüche differenziert ist, wobei die Zelle ein dauerhaft vererbbares Expressionskonstrukt umfasst, das für die Expression eines Transgens in Stammzellen geeignet ist, wobei das Konstrukt umfasst:
- mindestens einen CAG-Promotor, wobei die Nukleotid-Sequenz des CAG-Promotors der in der SEQ ID Nr. 1 gegebenen Sequenz entspricht, und der Promotor sowohl in Stammzellen als auch in differenzierten Geweben funktionsfähig ist, wobei der Promotor konstitutiv ist während die Expressions-Ebene hiervon einer Geweberegulation oder zelltypspezifischen Regulation in Kardiomyozyten unterliegt, und
- ein Transgen, das unter der Kontrolle des Promotors ist, wobei das Transgen in der Stammzelle exprimiert wird.

7. Differenzierte Zelle nach Anspruch 6, wobei die Zelle ein Kardiomyozyten ist.

8. Verfahren zur Herstellung einer differenzierten Stammzelle *in vitro,*
bei dem die differenzierte Stammzelle ein dauerhaft vererbbares Expressionskonstrukt umfasst, das für die Expression eines Transgens in Stammzellen geeignet ist, wobei das Konstrukt umfasst
- mindestens den sich zweifach auszeichnenden CAG-Promotor, wie in Anspruch 1 definiert, und
- ein Transgen, das unter der Kontrolle des Promotors ist;
wobei das Verfahren umfasst
i) Bereitstellen eines Konstrukts, welches umfasst
- mindestens den CAG-Promotor, wie in Anspruch 1 definiert, und
- ein Transgen, das unter der Kontrolle des Promotors ist, wobei das Transgen in der Stammzelle exprimiert wird,
ii) Einbringen des in Schritt i) definierten Konstrukts in eine tierische oder menschliche Stammzelle und,
iii) Differenzieren der Stammzelle.

9. Verfahren nach Anspruch 8, wobei das Einbringen des Konstrukts in Schritt ii) durch virale Transduktion oder durch Verwendung eines Transposon-Transposase-Systems durchgeführt wird.

10. Verwendung der Stammzellen nach einem der Ansprüche 1 bis 5, um den Effekt einer Testverbindung auf die Stammzellen-Differenzierung zu testen.

11. Reagenzien-Kit, umfassend:
- Eine Stammzelle, wie in einem der Ansprüche 1 bis 5 definiert, und
- eine oder mehrere Testreagenzien, die die Umsetzung der Verwendung gemäß Anspruch 10 ermöglichen.

## Revendications

1. Une cellule souche d'animal ou d'humain contenant une copie d'une construction d'expression stable héréditaire qui est appropriée pour l'expression de transgènes dans les cellules souches, ladite construction comprenant:
- au moins un promoteur CAG dans lequel la séquence nucléotide dudit promoteur CAG est la séquence donnée dans SEQ ID NO. 1, ledit promoteur étant opérable à la fois dans les cellules souches ainsi que les tissus différenciés, dans lequel ledit promoteur est constitutif pendant que le niveau d'expression de celui-ci étant sujet au tissu ou d'un règlement spécifique du type cellulaire dans les cardiomyocytes, et
- sous le contrôle dudit promoteur, un transgène, dans lequel ledit transgène est exprimé dans ladite cellule souche.

2. La cellule souche selon la revendication 1, qui est une cellule souche embryonnaire humaine (HUES).

3. La cellule souche selon la revendication 1, qui est une cellule souche embryonnaire animale.

4. La cellule souche selon l'une quelconque des revendications précédentes, dans laquelle ladite construction d'expression comprend en outre des séquences sélectionnées à partir d'un des groupes suivant :
- des séquences virales de transduction,
- des séquences de transposon dérivés,
- une paire de séquences permettant une recombinaison spécifique de site en amont du promoteur et en aval dudit transgène.

5. La cellule souche selon l'une quelconque des revendications précédentes, dans laquelle ledit transgène est choisi parmi :
- un gène rapporteur et protéine rapporteuse codée par ledit gène rapporteur est exprimée dans ladite cellule souche,
- une construction pour l'expression de siRNA et un gène rapporteur connecté, et ledit siRNA et la protéine rapporteuse codée par ledit gène rapporteur sont exprimés dans ladite cellule souche ou dans ces dérivés différenciés,
- un gène codant pour une protéine fluorescente choisie parmi un groupe comprenant la protéine fluorescente verte (GFP), la protéine fluorescente rouge (RFP), la protéine fluorescente jaune (YFP), la protéine fluorescente cyan (CFP), la protéine fluorescente DS-redMST ; la luciférase ; la β-Galactosidase; et
- un gène codant pour une protéine qui fournit une résistance aux agents chimiques sélectionnés, préférentiellement à la puromycine, néomycine ou autres agents cytotoxiques.

6. Une cellule différente de la cellule souche selon l'une quelconque des revendications précédentes, dans laquelle la cellule comprend une construction d'expression héréditaire stable qui est adaptée pour l'expression d'un transgène dans les cellules souches, ladite construction comprenant :
- au moins un promoteur CAG dans lequel la séquence nucléotide dudit promoteur CAG est la séquence donnée dans SEQ ID Nr., ledit promoteur étant opérable à la fois dans les cellules souches et les tissus différenciés, dans lequel ledit promoteur est constitutif pendant que le niveau d'expression de celui-ci étant sujet au tissu ou d'un règlement spécifique du type cellulaire dans les cardiomyocytes, et
- sous le contrôle dudit promoteur, un transgène, dans lequel ledit transgène est exprimé dans ladite cellule souche.

7. La cellule différenciée selon la revendication 6, dans laquelle ladite cellule est une cardiomyocyte.

8. Procédé pour la synthèse *in vitro* d'une cellule souche différenciée
ladite cellule souche comprenant
une construction d'expression héréditaire stable qui est adaptée pour l'expression d'un transgène dans les cellules souches, ladite construction comprenant
- au moins la double caractéristique CAG promoteur comme défini selon la revendication 1 et,
- un transgène sous le contrôle dudit promoteur;
ledit procédé comprenant
i) fournissant une construction qui comprend
- au moins le promoteur CAG comme défini selon la revendication 1, et,
- sous le contrôle dudit promoteur, un transgène, dans lequel ledit transgène est exprimé dans ladite cellule souche,
ii) introduire la construction définie dans l'étape i) dans une cellule souche animale ou humaine et,
iii) différencier ladite cellule souche.

9. Procédé selon la revendication 8, dans lequel
- ladite introduction de ladite construction selon ii) est réalisée par transduction virale ou par l'utilisation d'un système transposon-transposase.

10. Utilisation des cellules souches selon l'une quelconque des revendications 1 à 5 pour tester l'effet d'un composé d'essai sur la différenciation des cellules souches.

11. Un kit réactif, comprenant:
- une cellule souche définie selon l'une quelconque des revendications 1 à 5, et
- un ou plusieurs réactifs d'essai permettant la mise en oeuvre de l'utilisation selon la revendication 10.
